# EUROPÄISCHE PATENTSCHRIFT

(11) **EP 3 176 714 B1**
(45) Veröffentlichungstag und Bekanntmachung des Hinweises auf die Patenterteilung: **29.01.2025**
(21) Anmeldenummer: 15197225.4
(22) Anmeldetag: 01.12.2015
(51) Int. Cl.: H04L 9/40, G16H 30/20, G16H 80/00, H04L 67/12, G06F 21/62, A61B 6/00, G16H 10/60

(54) **GESICHERTES TEILEN VON MEDIZINISCHEN BILDERN**
SECURE SHARING OF MEDICAL IMAGES
ÉLÉMENTS SÉCURISÉS D'IMAGES MÉDICALES

(43) Veröffentlichungstag der Anmeldung: 07.06.2017
(73) Patentinhaber: Siemens Healthineers AG, 91301 Forchheim (DE)
(72) Erfinder: Ukis, Vladyslav, 90489 Nürnberg (DE)
(74) Vertreter: Siemens Healthineers Patent Attorneys

(56) Entgegenhaltungen:
- DE-A1- 102013 211 540
- DE-A1- 102014 003 889
- US-A1- 2009 112 629
- US-A1- 2011 016 328
- US-A1- 2011 110 568
- US-A1- 2012 046 972
- US-A1- 2013 208 966

## Beschreibung

Die vorliegende Erfindung betrifft ein Verfahren zur gesicherten Kollaboration auf der Basis von medizinischen Bildern, ein System, eine Befehlseinheit und eine Pseudonymisierungseinheit sowie ein Computerprogramm.

Die Erfindung liegt auf den Gebieten der Medizintechnik und der Sicherheitstechnik für Cloud-basierte Architekturen.

Auf dem Gebiet der Medizin ist es heute wichtig, dass bei lebensentscheidenden Fragen mehrere Experten (zum Beispiel im Rahmen eines interdisziplinären Konzils) medizinische Bilder sichten können. Eine Cloud-basierte Architektur ermöglicht dies.

Im Stand der Technik sind hierzu unterschiedliche Cloud-basierte Dienste (image sharing services) bekannt, die es ermöglichen, dass Bilder zwischen Krankenhausabteilungen und zwischen verschiedenen Kliniken und Forschungseinrichtungen, die entfernt voneinander lokalisiert sind, geteilt werden. Dies hat den Vorteil, dass eine Bildstudie an einen anderen Arbeitsplatz zur dortigen Sichtung versendet werden kann. Bei der Sichtung erweist es sich in der Praxis jedoch häufig notwendig, dass Vorgängerstudien des Patienten erforderlich sind, um eine abschließende Aussage oder eine grundlegende Entscheidung für weitere Aktionen treffen zu können. Die Vorgängerstudien können allerdings aufgrund von Sicherheitserfordernissen nicht auf einem entfernten System angezeigt werden. Dies liegt hautsächlich daran, dass in den Bildern auch sicherheitskritische Daten enthalten sind, von denen Rückschlüsse auf die Person des Patienten gezogen werden könnten.

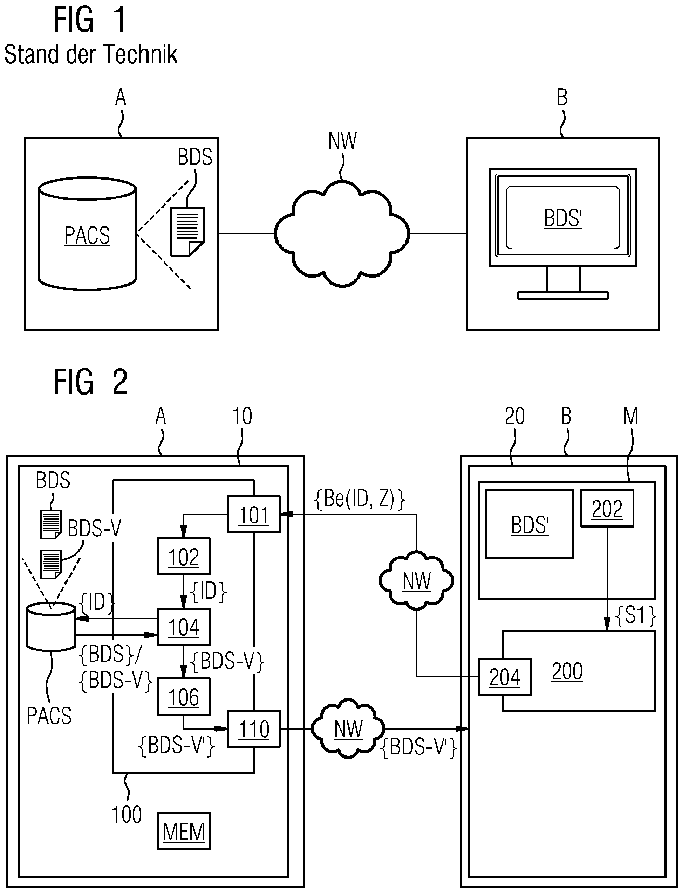

Dieses Szenario aus dem Stand der Technik ist in Figur 1 widergegeben. Ein Arzt eines ersten Krankenhauses A möchte, Bilder, die in einem PACS-System gespeichert sind, mit einer Person teilen, die in einem zweiten Krankenhaus B arbeitet, um sie dort auf einem Arbeitsplatz zur Anzeige zu bringen. In den bekannten Systemen nach dem Stand der Technik werden dazu die jeweiligen Bilddaten anonymisiert oder derart verändert, dass keine personenidentifizierende Angaben mehr in dem Bild enthalten sind. Sollen jetzt auf dem Arbeitsplatz des Krankenhauses B Vorgängerstudien angezeigt werden, so ist dies ohne Verstoß gegen Sicherheitsregelungen nicht möglich.

Die DE 10 2014 003 889 A1 betrifft ebenfalls die anonymisierte Bereitstellung von medizinischen Bildern. Hier geht es um das Verfügbarmachen von Bildern für einen Patienten, indem ein Link (URL) an den Patienten gesendet wird, der auf eine Adresse auf einem Web-Server verweist, der über das Internet zugreifbar ist und unter dem nur die reinen Bilddaten ohne PHI-Informationen abgelegt sind. Über diesen Link kann der Patient dann auf seine Daten zugreifen. Ein Nachfordern von Bildern zu bereits angezeigten Bildern ist hier nicht beschrieben.

Die DE 10 2013 211 540 A1 betrifft eine kontext-abhängige Pseudonymisierung von sicherheitskritischen Medizindaten. Diese Druckschrift hat zum Ziel, die Art der Pseudonymisierung an die jeweils sich anschließende Verarbeitung anzupassen, um die verfügbare Netzwerkbandbreite möglichst optimal ausnutzen zu können. Ein Nachfordern von Bildern zu bereits angezeigten Bildern ist auch hier nicht beschrieben.

Der vorliegenden Erfindung liegt die Aufgabe zugrunde, ein System zum Teilen von medizinischen Bildern dahingehend zu verbessern, dass zu einem geteilten Bild Vorgängerstudien angefordert werden können. Des Weiteren soll die Sicherheit bei der Kollaboration im medizinischen Bereich verbessert werden. Diese Aufgabe wird jeweils durch einen Gegenstand nach den unabhängigen, nebengeordneten Ansprüchen gelöst. Vorteilhafte Ausführungsformen sind Gegenstand der abhängigen Ansprüche, der Beschreibung und der Zeichnungen.

Gemäß einem ersten Aspekt wird die Aufgabe gelöst durch ein System zum gesicherten Nachladen von Vorgängerstudien zu einer Bilddatenstudie auf einem Empfängerknoten, der entfernt von einem Bildsendeknoten angeordnet ist, mit:
- Einer Befehlseinheit und einem Signalsensor auf einem Empfängerknoten, wobei der Signalsensor zeitgleich mit der gerade dargestellten Bilddatenstudie angezeigt wird und dazu bestimmt ist, ein Bildanforderungssignal zu der gerade dargestellten Bilddatenstudie zu erfassen und wobei die Befehlseinheit nach Erfassen des jeweiligen Bildanforderungssignals dazu bestimmt ist, einen Befehl zu erzeugen und an eine Pseudonymisierungseinheit zu senden, wobei der Befehl Steueranweisungen enthält, um zu der gerade dargestellten Bilddatenstudie eine Vorgängerstudie von dem Bildsendeknoten zu laden,
- Der Pseudonymisierungseinheit, die auf dem Bildsendeknoten angeordnet ist und folgende elektronische oder computerbasierte Module umfasst:
   - Einen Extraktor, der dazu bestimmt ist, auf Empfang des Befehls einen Identifikationsdatensatz aus dem Befehl zu extrahieren
   - Einen Lader, der dazu bestimmt ist, auf Basis des extrahierten Identifikationsdatensatzes auf ein Bildspeichersystem zum Laden der Vorgängerstudie zuzugreifen
   - Einen Pseudonymisierungsprozessor, der dazu bestimmt ist, zu der geladenen Vorgängerstudie einen pseudonymisierten Bilddatensatz zu erzeugen,
   - Eine Empfängerknoten-Schnittstelle, die dazu bestimmt ist, den pseudonymisierten Bilddatensatz an den Empfängerknoten oder an einen alternativen Empfängerknoten zu senden.

Bei der Bilddatenstudie handelt es sich um zumindest ein Bild oder einen Bilddatensatz, der von einer medizinischen bildgebenden Modalität (Computertomographen, wie Dual Source CT Scanner oder Single Source CT Scanner, Magnetresonanztomographen, Röntgengeräte etc.) erfasst worden ist. Er liegt vorzugsweise in einem standardisierten Format (DICOM-Format) vor. Die Bilddatenstudie ist einem bestimmten Patienten zugeordnet. Üblicherweise werden für einen Patienten in einem gewissen Zeitraum mehrere Bildstudien angefertigt. So gibt es in der Regel zu einer Bildstudie Vorgängerstudien (so genannte prior studies).

Die Vorgängerstudie ist ein Bilddatensatz von demselben Patienten, vorzugsweis zu einem anderen Untersuchungszeitpunkt. Es ist jedoch auch möglich, dass sich die Vorgängerstudie auf denselben oder einen anderen Untersuchungszeitpunkt wie die Bildstudie bezieht, allerdings von einer anderen Modalität erfasst worden ist (z.B. MR anstatt CT). In Weiterbildungen der Erfindung kann die Vorgängerstudie auch eine Referenzstudie mit demselben im Bild repräsentierten Zielvolumen sein. Ebenso ist es möglich, dass eine Bildstudie von einem zurückliegenden (nicht aktuellen) Zeitraum auf dem Empfängerknoten geteilt und zur Anzeige gebracht werden soll und dass keine Vorgängerstudie in dem Sinne, sondern eine Studie zu einem späteren Zeitpunkt angefordert wird, also eine Nachfolgestudie. Dies liegt ebenso im Rahmen der Erfindung.

Der Bildsendeknoten steht üblicherweise direkt, über ein gesichertes Netzwerk in Datenaustausch mit einem Bildspeichersystem, das als zentrales Datenarchiv zur Speicherung von Bilddaten bestimmt ist. Die Verbindung zwischen Bildspeichersystem, das als PACS System ausgebildet sein kann und dem Bildsendeknoten mit der Pseudonymisierungseinheit ist ein gesichertes lokales Netzwerk (z.B. ein local area network, LAN) .

Der Begriff "Nachladen" bezieht sich auf die hauptsächliche Ausführungsform der Erfindung, bei der die Vorgängerstudie auf eine Bilddatenstudie bezogen ist oder dieser zugeordnet ist, die gerade auf dem Empfängerknoten angezeigt wird. Die Bilddatenstudie ist somit auf dem Empfängerknoten geladen und wird dort angezeigt und zu dieser angezeigten Studie soll die Vorgängerstudie nachgeladen werden.

Grundsätzlich umfasst das System zwei unterschiedliche computerbasierte Knoten oder Rechnersysteme (Netzwerke mit Prozessorkernels in realer oder virtueller Form):
- Einen Empfängerknoten auf dem die Bilddatenstudie angezeigt (zur Kollaboration geteilt) werden soll und
- Einen Bildsendeknoten, der in Datenaustausch mit einem Bildspeichersystem steht und der als Sendeinstanz fungiert und von dem aus die Bilddatenstudien geteilt werden soll.

Beide Knoten interagieren über ein Netzwerk, insbesondere über eine öffentliche, ungesicherte Netzwerkverbindung (z.B. das Internet). In Folgenden wird die Erfindung der Einfachheit halber in der Ausführungsform beschrieben, bei der ein Bilddatensatz von dem Bildsendeknoten an nur einen Empfängerknoten zur Kollaboration gesendet werden soll. Es liegt jedoch ebenso im Rahmen der Erfindung, dass eine oder mehrere Bildstudien parallel an mehrere Empfängerknoten gesendet werden, insbesondere nach einem Broadcasting Prinzip. Auch kann ein Empfängerknoten Bilddatensätze von unterschiedlichen Bildsendeknoten empfangen. Es ist auch möglich, dass das Bildanforderungssignal mehrere Parameter umfasst, in denen z.B. spezifiziert werden kann, dass eine anzufordernde Vorgängerstudie nicht auf dem aktuellen gegenwärtig bestimmten Empfängerknoten geladen werden soll, sondern auf einem anderen Knoten. In diesem Fall kann ein weiteres Eingabefeld vorgesehen sein, auf dem die Eingabe einer Adresse (z.B. einer IP-Adresse oder eines Namens) eingegebene werden kann.

Zur gesicherten Übertragung der Daten zwischen Bildsende- und Empfängerknoten müssen deshalb Sicherungsmaßnahmen vorgesehen werden, damit bei einem Abhören der Netzwerkverbindung auf Basis der übertragenen Daten kein Rückschluss auf die Identität des Patienten gezogen werden kann. Dabei ist zu berücksichtigen, dass die medizinischen Bilddatensätze in der Regel sicherheitskritischen Inhalt umfassen, der zugriffsgeschützt zu verarbeiten und zu behandeln ist. So muss sichergestellt sein, dass Patienten-identifizierende Angaben (z.B. Name, Alter, Geschlecht, Wohnort etc.) nicht in Kombination mit Bilddaten oder Bilddatenausschnitten über die Grenzen der jeweiligen zugriffsgeschützten Umgebung hinaus gelangen. Diese zugriffsgeschützten Inhalte werden als "protected health information (PHI)" bezeichnet. Die Pseudonymisierungseinheit dient nun dazu, die PHI-Bestandteile aus dem jeweiligen Bilddatensatz zu entfernen. Vorzugsweise wird eine Zuordnung zwischen dem originalen Bilddatensatz (mit PHI Daten) und dem pseudonymisierten Bilddatensatz (ohne PHI Daten) gespeichert. Vorzugsweise wird folgende Zuordnung gespeichert: ein Identifikator zur originalen Bilddatenstudie (im Folgenden auch kurz: Studie), ein Identifikator zur pseudonymisierten Studie, ein Identifikator zu den PHI-Daten des jeweiligen Patienten im Original und den Identifikationsdaten zu den PHI-Daten nach der Pseudonymisierung.

Die Erzeugung des pseudonymisierten Bilddatensatzes kann je nach Ausführungsform der Erfindung nach unterschiedlichen Methoden erfolgen. Die pseudonymisierten Bilddaten kennzeichnen sich dadurch, dass alle den Patienten identifizierenden Abschnitte oder Anteile manipuliert oder aus dem Datensatz entfernt worden sind. Die Bilddaten können mit unterschiedlichen Maßnahmen de-identifiziert werden, so dass diese keine (den Patienten) identifizierenden Hinweise mehr enthalten. Dazu können gezielt und dezidiert vorbestimmbare Kenngrößen aus dem Datensatz eliminiert, generalisiert, ersetzt oder anderweitig manipuliert werden, wie z.B. Name, geographische Daten, Kontaktnummern oder Adressen, darunter auch Email-Adressen, Sicherheitsnummern, sonstige ID-Nummern (z.B. von Gesundheitskarten oder Bankkarten etc.), biometrische Daten, Fotos etc. Eine alternative Maßnahme besteht in der Anwendung von statistischen Verfahren, die darauf basieren, die statistische Wahrscheinlichkeit zu prüfen, ob aus dem de-identifizierten Datensatz wieder die Originaldaten herleitbar sind. Alternativ kann hier auch eine Anonymisierung der PHI-Daten eingesetzt werden, die in der Regel keine zentrale Zuordnungsinstanz zwischen Original PHI-Daten und Pseudonym vorhält. Die anonymisierten oder pseudonymisierten Bilddaten liegen grundsätzlich sichtbar und für jeden lesbar (als Plaintext)- also unverschlüsselt - vor.

Das Bildaufforderungssignal ist vorzugsweise ein Signal, das über eine Bedienung eines Eingabefeldes auf einer (vorzugsweise graphischen) Benutzeroberfläche eingegeben werden kann. Alternativ kann das Bildaufforderungssignal jedoch auch ein anders optisches oder akustisches Signal sein.

Das von der Befehlseinheit empfangene oder erfasste Bildaufforderungssignal dient dazu, einen Befehl zu erzeugen. Der Befehl enthält Steueranweisungen, um zu der angezeigten Bildstudie eine Vorgängerstudie von dem Bildsendeknoten zu laden. Der Befehl enthält einen Identifikationsdatensatz, der die angezeigte Studie und/oder die gewünschten Vorgängerstudie identifiziert. Des Weiteren kann der Befehl noch weitere Kriterien umfassen, z.B. welche der verfügbaren Vorgängerstudien genau geladen werden soll. Dies kann über eine Spezifikation des Zeitraums, der Modalität, der Lokalisation der jeweiligen Anatomie oder über andere medizinische Parameter erfolgen.

Ein wesentlicher Vorteil der Erfindung ist darin zu sehen, dass die jeweiligen pseudonymisierten Datensätze nur im Bedarfsfall und auf Anforderung erzeugt werden, also, wenn eine Studie geteilt werden soll oder wenn der Empfänger der geteilten Studie Vorgängerstudien anfordert. Dies verringert den Ressourceneinsatz zur Speicherung und zur Berechnung von pseudonymisierten Datensätzen deutlich.

Gemäß einer vorteilhaften Ausführungsform der Erfindung umfasst die Pseudonymisierungseinheit ein Pseudonymisierungs-Frontend und ein Pseudonymisierungs-Backend. Das Pseudonymisierungs-Frontend ist auf dem Bildsendeknoten und das Pseudonymisierungs-Backend entfernt angeordnet. Das Backend kann insbesondere auf einem Netzknoten installiert sein.

Gemäß einer weiteren, vorteilhaften Ausführungsform der Erfindung umfasst die Befehlseinheit ein Befehls-Frontend und ein Befehls-Backend, wobei das Befehls-Frontend auf dem Empfängerknoten und das Befehls-Backend auf einer entfernten Instanz und insbesondere auf einem Netzknoten angeordnet sind.

Die Befehlseinheit und die Pseudonymisierungseinheit interagieren über ein digitales Netzwerk, insbesondere über ein öffentliches, unsicheres Netzwerk. Deshalb werden erfindungsgemäß auch keine Bilddaten mit PHI-Informationen übertragen, sondern nur pseudonymisierte Bilddatensätze ohne PHI-Daten.

Das Backend der Befehlseinheit und/oder das Backend der Pseudonymisierungseinheit können als Webservices ausgebildet sein und auf einem Server gehostet sein, der über das Internet zugreifbar ist. In einer Ausführungsform der Erfindung ist das Backend der Befehlseinheit und das Backend der Pseudonymisierungseinheit in unterschiedlichen Cloud-Systemen gehostet.

Gemäß einem Aspekt betrifft die Erfindung eine Befehlseinheit zum Betrieb in einem vorstehend beschriebenen System und insbesondere zum Betrieb in einem Empfängerknoten. Die Befehlseinheit umfasst einen Signalsensor, der zeitgleich mit der gerade dargestellten Bilddatenstudie angezeigt wird und dazu bestimmt ist, ein Bildanforderungssignal zu der gerade dargestellten Bilddatenstudie zu erfassen und wobei die Befehlseinheit nach Erfassen eines Bildanforderungssignals dazu bestimmt ist, einen Befehl zu erzeugen und über eine Pseudonymisierungseinheit-Schnittstelle zu versenden. Der Signalsensor kann zur Erfassung von Eingabesignalen eines Anwenders und insbesondere als Eingabefeld auf einer graphischen Benutzeroberfläche ausgebildet sein. Wenn der Anwender dann in diesem Eingabefeld ein Häkchen oder Kreuz setzt, dann gilt dies als Signal dafür, dass eine Vorgängerstudie aus dem entfernten Bildspeichersystem angefordert wird. In einer bevorzugten Weiterbildung der Erfindung können zu dem Bildaufforderungssignal noch weitere Parameter erfasst werden, z.B. um eine spezielle Vorgängerstudie aus einer Menge von verfügbaren Vorgängerstudien zu spezifizieren oder um den Zeitpunkt des Ladens auf dem Empfängerkonten zu spezifizieren oder um einen oder mehrere weitere Empfängerknoten zu bestimmen, auf denen die Vorgängerstudie ebenfalls angezeigt werden soll.

Gemäß einem weiteren Aspekt betrifft die Erfindung eine Pseudonymisierungseinheit zum Betrieb in einem oben beschriebenen System. Die Pseudonymisierungseinheit steht in Datenaustausch mit einem Bildspeichersystem, insbesondere über ein internes gesichertes Netzwerk (z.B. LAN). Die Pseudonymisierungseinheit umfasst folgende elektronische oder computerbasierte Module:
- Einen Extraktor, der dazu bestimmt ist, auf Empfang eines Befehls einen Identifikationsdatensatz aus dem Befehl zu extrahieren. In Bezug auf den Befehl und den Identifikationsdatensatz gilt das unten zur Beschreibung des Verfahrens Gesagte entsprechend.
- Einen Lader, der dazu bestimmt ist, auf Basis des extrahierten Identifikationsdatensatzes auf das Bildspeichersystem zum Laden einer oder mehrere Vorgängerstudie(n) zuzugreifen.
- Einen Pseudonymisierungsprozessor, der dazu bestimmt ist, zu der geladenen Vorgängerstudie einen pseudonymisierten Bilddatensatz oder entsprechend mehrere Datensätze zu erzeugen.
- Eine Empfängerknoten-Schnittstelle, die dazu bestimmt ist, den pseudonymisierten Bilddatensatz an den Empfängerknoten oder an einen alternativen Empfängerknoten zu senden.

Gemäß einem weiteren Aspekt betrifft die Erfindung ein Verfahren zum gesicherten Nachladen von Vorgängerstudien zu einer Bilddatenstudie auf einem Empfängerknoten, der entfernt von einem Bildsendeknoten angeordnet ist, wobei das Verfahren folgende Verfahrensschritte umfasst:
- Anzeigen eines Signalsensors zeitgleich mit der gerade dargestellten Bilddatenstudie
- Erfassen eines Bildanforderungssignals auf dem Signalsensor des Empfängerknotens.
- Erzeugen eines Befehls, wobei der Befehl Steueranweisungen enthält, um zu der gerade dargestellten Bilddatenstudie eine Vorgängerstudie von dem Bildsendeknoten zu laden und Versenden des Befehls an eine Pseudonymisierungseinheit, wobei der Befehl vorzugsweise einen Identifikationsdatensatz umfasst. Alternativ kann der Identifikationsdatensatz auch über einen zusätzlichen Kommunikationskanal eingelesen und bereitgestellt werden. Der Identifikationsdatensatz identifiziert den Patienten und/oder die Bildstudie des Patienten eineindeutig.
- Empfangen des Befehls auf der Pseudonymisierungseinheit.
- Extrahieren eines Identifikationsdatensatz aus dem empfangenen Befehl.
- Laden zumindest einer Vorgängerstudie aus dem lokalen Bildspeichersystem auf Basis des extrahierten Identifikationsdatensatzes.
- Erzeugen zumindest eines pseudonymisierten Bilddatensatzes zu der/den Vorgängerstudie(n).
- Versenden des erzeugten pseudonymisierten Bilddatensatzes über das Netzwerk an den Empfängerknoten und/oder an weitere spezifizierte Empfängerknoten.

Das Verfahren umfasst mehrere Verfahrensschritte, die auf unterschiedlichen Entitäten ausgeführt werden, nämlich auf dem Bildsendeknoten und auf dem Empfängerknoten. Vorzugsweise werden die ersten beiden Verfahrensschritte auf dem Empfängerkonten und die restlichen auf dem Bildsendeknoten ausgeführt. Für den Fachmann ist es jedoch ersichtlich, dass hier auch zusätzliche Knoten eingeschaltet und zwischengeschaltet werden können.

In einer bevorzugten Ausführungsform der Erfindung wird eine Zuordnung zwischen dem (originalen) Bilddatensatz, Identifikationsdatensatz und/oder pseudonymisierten Bilddatensatz gespeichert wird. Diese Zuordnung wird vorzugsweise lokal auf der Pseudonymisierungseinheit gespeichert, also auf dem System auf dem auch der Bildspeicher angeordnet ist und/oder auf dem ein pseudonymisierter Datensatz erstellt wird.

In einer bevorzugten Ausführungsform der Erfindung ist es vorgesehen, dass das Bildanforderungssignal nur zu einem aktuell gerade angezeigten Bilddatensatz eingegeben werden kann, um automatisch eine Zuordnung zu der Bildstudie zu generieren. Dies erfolgt, indem auf einer Bildschirmoberfläche des Empfängerknotens ein Eingabefeld nur während der Darstellung eines Bilddatensatzes bereitgestellt wird. Wird dann ein Bildanforderungssignal auf dem Signalsensor erfasst, so ist zugleich definiert, für welchen Bilddatensatz eine Vorgängerstudie angefordert wird. Andernfalls kann das Bildanforderungssignal auch unabhängig von der Anzeige einer geteilten Bildstudie eingegeben werden. In diesem Fall werden Definitionsmittel bereitgestellt, mit denen die Bildstudie identifiziert werden kann, z.B. über ein weiteres Eingabefeld.

Die Pseudonymisierungseinheit wird vorzugsweise im Passiv-Modus betrieben. Das heißt, dass sie nur auf Erhalt eines Befehls der Befehlseinheit des Empfängerknotens aktiv wird. Nur auf einen Anforderungsbefehl für weitere Vorgängerstudien wird die Pseudonymisierungseinheit aktiviert und erstellt einen pseudonymisierten Bilddatensatz für die zuvor geteilten Bilddaten. Dies hat den Vorteil, dass das Verfahren sehr prozessor-effektiv betrieben werden kann, indem nicht unnötig Pseudonymisierungen berechnet, gespeichert und/oder versendet werden müssen. Alternative Ausführungsformen der Erfindung sehen einen Aktiv-Modus zum Betrieb der Pseudonymisierungseinheit vor, bei dem ereignisbasiert bei Speicherung einer neuen Studie bzw. eines neuen Bilddatensatzes im Speicher immer zugleich auch ein pseudonymisierter Bilddatensatz erzeugt und gespeichert wird, der dann für Nachforderungen unmittelbar nachgeladen werden kann.

Gemäß einem anderen Aspekt wird die Erfindung gelöst durch einen Bildsendeknoten mit einer Pseudonymisierungseinheit, wie oben beschrieben.

Gemäß einem anderen Aspekt wird die Erfindung gelöst durch einen Empfängerknoten mit einer Befehlseinheit, wie oben beschrieben.

Bei der vorstehenden Beschreibung der Erfindung mit deren unterschiedlichen Aspekten und Ausführungsformen können erwähnte Merkmale, Vorteile und/oder alternative Ausführungsformen ebenso auch auf die anderen beanspruchten Gegenstände übertragen werden und umgekehrt. Mit anderen Worten können auch die gegenständlichen Ansprüche (die beispielsweise auf ein System oder auf ein Computerprogrammprodukt gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

Eine weitere Aufgabenlösung besteht in einem Computerprogrammprodukt, das in einen Speicher eines Computers geladen oder ladbar ist mit Computerprogrammcode zur Durchführung des oben näher beschriebenen Verfahrens, wenn das Computerprogrammprodukt auf dem Computer ausgeführt wird.

Eine weitere Aufgabenlösung sieht ein Computerprogramm vor, mit Computerprogrammcode zur Durchführung aller Verfahrensschritte des oben näher beschriebenen Verfahrens, wenn das Computerprogramm auf einem Computer ausgeführt wird. Dabei ist es auch möglich, dass das Computerprogramm auf einem von einem Computer lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: eine schematische Übersichtsdarstellung eines Systems zum Teilen von Bilddaten nach dem Stand der Technik
- Fig. 2: ein Blockschaltbild eines Systems gemäß einer bevorzugten Ausführungsform der Erfindung;
- Fig. 3: ein Blockschaltbild eines Systems gemäß einer bevorzugten Ausführungsform der Erfindung in einer Übersichtsdarstellung und
- Fig. 4: ein Ablaufdiagramm für Verfahrensschritte eines Verfahrens gemäß einer bevorzugten Ausführungsform der Erfindung die auf verteilen Einheiten des Systems ausgeführt werden.

### Detaillierte Beschreibung der Figuren

Im Folgenden wird die Erfindung in Zusammenhang mit Figur 2 näher beschrieben.

In einer ersten Klinik A möchte ein Arzt für die Einleitung weitere medizinischer Maßnahmen für einen Patienten, eine bestimmte Bilddatenstudie BDS, die lokal in einem Bildarchivierungssystem PACS gespeichert ist mit seinem Kollegen teilen, der in einer entfernten zweiten Klinik B arbeitet. Allerdings können die beiden Einheiten bzw. Kliniken A, B Daten nur über ein ungesichertes Netzwerk NW, z.B. das Internet, austauschen. Um Sicherheitsanforderungen zu genügen, ist es deshalb gefordert, dass Bilddatenstudien BDS grundsätzlich nur in pseudonymisierter oder anonymisierter Weise über das ungesicherte Netzwerk NW übertragen werden, also ohne den Patienten identifizierende PHI Daten (PHI: Protected Health Information) .

Dazu werden die zu teilenden Bilddatenstudien BDS auf einem Produzentensystem, dem Bildsendeknoten 10 (Klinik A in Figur 1), pseudonymisiert und als pseudonymisierter Bilddatensatz BDS` an einen Empfängerknoten 20 (Klinik B in Figur 1) übertragen und dort auf einem Monitor M dargestellt. Der Kollege kann den pseudonymisierten Bilddatensatz BDS` nun ebenfalls einsehen und die weiteren Maßnahmen mit seinem Kollegen in Klinik A besprechen. Stellt sich dabei heraus, dass der Kollege an dem Empfängerknoten 20 noch Vorgängerstudien benötigt, um eine Aussage treffen zu können, so war es im Stand der Technik bislang nicht möglich, das Nachladen von Vorgängerstudien zu der bereits geteilten und angezeigten Studie auf dem Empfängerknoten 20 auszulösen.

Die Erfindung schlägt nur vor, auf dem Empfängerknoten 20 neben dem Monitor M zur Anzeige des geteilten pseudonymisierten Bilddatensatzes BDS` eine Befehlseinheit 200 bereitzustellen. Die Befehlseinheit 200 steht in Datenaustausch mit einem Signalsensor 202, der dazu dient, ein Bildanforderungssignal S1 zu erfassen. Das Bildanforderungssignal S1 kann über eine Anwenderaktion (wie z.B. das Betätigen einer Schaltfläche auf der Benutzeroberfläche und/oder über eine andere Eingabeeinrichtung) erfasst und an die Befehlseinheit 200 weitergeleitet werden. Diese berechnet dann daraus einen Befehl Be. Der Befehl Be umfasst einen Identifizierungsdatensatz ID, der die nachzuladende Studie identifiziert und/oder die gerade dargestellte Studie BDS` zu der eine Vorgängerstudie geladen werden soll. Dies kann in einer bevorzugten Ausführungsform der Erfindung erfolgen, indem der Signalsensor 202 zusammen (zeitgleich) mit der geteilten Bilddatenstudie BDS` auf dem Monitor M angezeigt wird. Dann kann automatisch eine Zuordnung des über den Signalsensor 202 erfassten Bildanforderungssignals S1 und der jeweils angezeigten Bilddatenstudie BDS` erfasst werden. Andernfalls können entsprechende weitere Eingabefelder zur Verfügung gestellt werden, über die der Anwender sowohl eine Bilddatenstudie BDS` als auch eine Vorgängerstudie BDS-V spezifizieren kann.

Aus Datenschutzgründen werden erfindungsgemäß auf dem (externen) Empfängerknoten 20 ausschließlich Bilddaten angezeigt, geladen und/oder verarbeitet, die pseudonymisiert oder anonymisiert sind und somit keine persönlichen, den Patienten identifizierenden Datensätze umfassen. Die originalen Bilddatensätze mit den PHI-Daten sind nur lokal auf dem Bildsendeknoten bzw. auf dem lokalen System des Bildsendeknotens 10 verfügbar.

Vorzugsweise wird eine Zuordnung zwischen dem originalen Bilddatensatz BDS und dem diesem zugeordneten pseudonymisierten Bilddatensatz BDS` lokal im Bildspeichersystem PACS oder auf dem Bildsendeknoten 10 bzw. einer ihm zugeordneten Instanz gespeichert, so dass es auch möglich ist, das Verfahren iterativ anzuwenden. Dies bedeutet, dass es in einem ersten Schritt möglich ist, einen originalen Bilddatensatz BDS zu teilen (zur Anzeige auf dem Monitor M des Empfängerknotens 20). Dann wird in einem zweiten Schritt eine erste Vorgängerstudie BDS-V1 zu dem angezeigten Bilddatensatz BDS angefordert. Diese erste Vorgängerstudie BDS-V1 wird auf dem Bildsendeknotensystem ermittelt und erneut pseudonymisiert und in Form eines pseudonymisierten Bilddatensatzes BDS-V1' an den Empfängerknoten 20 gesendet. Soll nun in einem dritten Schritt eine zweite Vorgängerstudie BDS-V2 angefordert werden, so ist auch dies möglich. Dazu wird in einem weiteren Schritt die zweite Vorgängerstudie BDS-V2 auf dem Bildsendeknotensystem ermittelt und erneut pseudonymisiert und in Form eines pseudonymisierten Bilddatensatzes BDS-V2' an den Empfängerknoten 20 gesendet. Diese Prozedur kann beliebig oft wiederholt werden, da jeweils die Zuordnung zwischen der Ausgangsstudie und ihrer pseudonymisierten Form gespeichert wird. Alternativ oder kumulativ kann auch ein Identifizierungsdatensatz für die jeweilige Studie gespeichert werden.

Der erzeugten Befehl Be kann neben dem Identifizierungsdatensatz ID noch weitere Zusatzinformation Z umfassen, die z.B. zur weiteren Spezifikation der Vorgängerstudie BDS-V bestimmt sind (z.B. über die Modalität, Zeitraumangaben umfassend etc.). Der Befehl Be wird dann an eine Empfangseinheit 101 auf dem Bildsendeknoten 10 weitergeleitet. Ein Extraktor 102 extrahiert aus dem empfangenen Befehl Be den Identifizierungsdatensatz ID und gibt diesen an einen Lader 104 weiter, der dazu bestimmt ist, mit selbigem auf das Bildspeichersystem PACS zuzugreifen, um die Vorgängerstudie BDS-V in den Bildsendeknoten 10 zu laden. Ein Pseudonym-Prozessor 106 dient dazu, zu der geladenen Vorgängerstudie BDS-V eine pseudonymisierte Vorgängerstudie BDS-V` zu erzeugen und diese über eine Empfängerkonten-Schnittstelle 110 an den Empfängerknoten 20 zur Darstellung auf dem Monitor M zu senden. Da die Vorgängerstudie ebenfalls pseudonymisiert ist, kann sie ohne Verstoß gegen Sicherheitsvorschriften über ein unsicheres Netzwerk NW übertragen werden.

In einer bevorzugten Ausführungsform der Erfindung kann auf dem Bildsendeknoten 10 ein Speicher MEM bereitgestellt werden, auf dem eine Zuordnung zwischen dem originalen Bilddatensatz BDS, dessen pseudonymisierter Form BDS`, den PHI-Daten der Studie BDS und/oder zur Vorgängerstudie BDS-V und/oder dem Identifizierungsdatensatz ID gespeichert werden. Vorzugsweise wird folgende Zuordnung abgelegt:
- Ein Study Instance UID der originalen Bilddatenstudie BDS
- Ein Identifikator zur Study Instance UID nach der Pseudonymisierung
- Patienten Identifikator im Original, also insbesondere die PHI-Daten des Patienten
- Ein Identifikator zur Patienten ID nach der Pseudonymisierung.

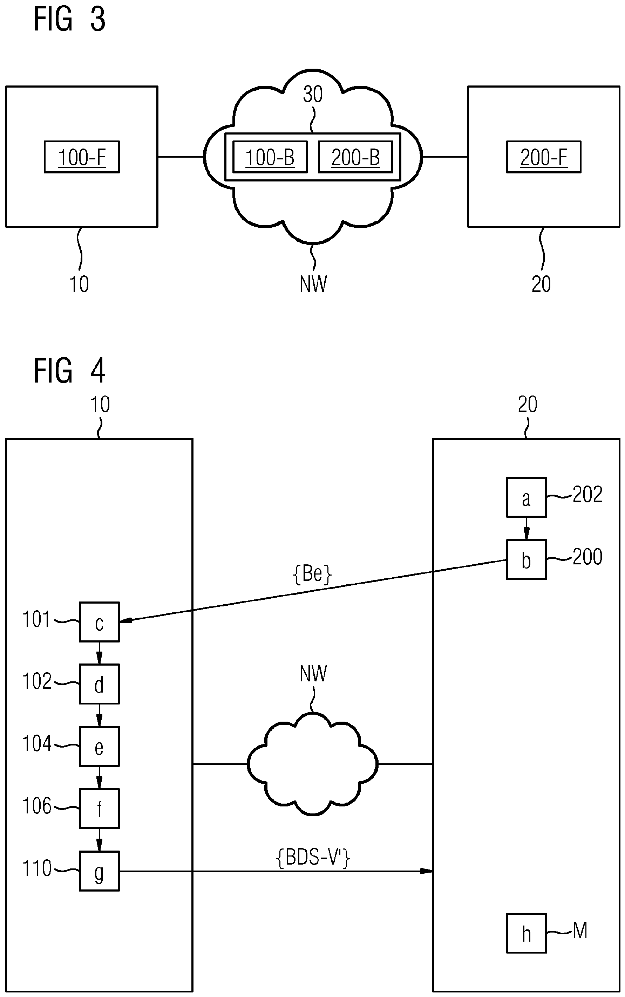

**Figur 3** zeigt, dass auf dem Bildsendeknoten 10 die Pseudonymisierungseinheit 100 und auf dem Empfängerkonten 20 die Befehlseinheit 200 installiert sind. Vorzugweise umfassen die beiden Einheiten 100, 200 jeweils ein Frontend und ein Backend. Das jeweilige Frontend kann als Browser-basierte Applikation in einem Fenster des jeweiligen Knotens geladen, bereitgestellt und/oder betrieben werden. Ein Pseudonymisierungs-Frontend 100-F ist auf dem Bildsendeknoten 10 installiert und ein Pseudonymisierungs-Backend 100-B kann entfernt installiert sein, so zum Beispiel auf einem entfernten Netzknoten 30. Ein Befehlseinheits-Frontend 200-F ist auf dem Empfängerknoten 20 installiert, während ein Befehlseinheits-Backend 200-B entfernt, z.B. ebenfalls auf demselben oder einem anderen Netzknoten 30 installiert sein kann.

**Figur 4** erklärt, welche Verfahrensschritte auf welchen computerbasierten Einheiten ausgeführt werden. In Schritt a wird ein Bildanforderungssignal S1 erfasst. Dies erfolgt vorzugsweise auf dem Signalsensor 202. In Schritt b wird auf Basis des Signals S1 ein Befehl Be erzeugt und an die Pseudonymisierungseinheit 100 versendet. In Schritt c wird der Befehl Be empfangen und in Schritt d wird aus diesem Befehl der Identifizierungsdatensatz ID extrahiert. In Schritt e kann dann auf Basis des extrahierten Identifizierungsdatensatzes ID auf das PACS zugegriffen werden, um die jeweilige Vorgängerstudie zu der bereits geteilten Studie herunterzuladen. In Schritt f wird der pseudonymisierte Vorgängerstudiendatensatz BDS-V` erzeugt, um anschließend in Schritt g an den Empfängerkonten 20 versendet zu werden. In Schritt h kann die Vorgängerstudie BDS-V` in pseudonymisierter Form auf dem Monitor M angezeigt werden. Die Schritte a, b und h werden auf dem Empfängerknoten 20 und die Schritte c bis g auf dem Bildsendeknoten 10 ausgeführt.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Für einen Fachmann ist es insbesondere offensichtlich, dass die Erfindung nicht nur für Bildstudien und Vorgängerstudien angewendet werden kann, sondern auch für andere medizinische Datensätze, die in unterschiedlichen Zeitphasen erfasst worden sind und nur in geschützter Form über ein öffentliches Netzwerk übertragen werden dürfen. Des Weiteren kann die Erfindung neben einer Pseudonymisierung auch mit anderen Verfahren zur Datensicherung von PHI-Daten betrieben werden. Auch können die Bauteile des Systems auf mehrere physikalische Produkte verteilt realisiert werden kann.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

## Patentansprüche

1. System zum gesicherten Nachladen von Vorgängerstudien (BDS-V) zu einer Bilddatenstudie (BDS) auf einem Empfängerknoten (20) von einem entfernten Bildsendeknoten (10), umfassend:
- Den Empfängerknoten (20) zur Darstellung von Bilddatenstudien, der eine Befehlseinheit (200) mit einem Signalsensor (202) umfasst, wobei der Signalsensor (202) zeitgleich mit der gerade dargestellten Bilddatenstudie (BDS`) angezeigt wird und dazu bestimmt ist, ein Bildanforderungssignal (S1) zu der gerade dargestellten Bilddatenstudie (BDS') zu erfassen und wobei die Befehlseinheit (200) nach Erfassen des jeweiligen Bildanforderungssignals (S1) dazu bestimmt ist, einen Befehl (Be) zu erzeugen und an eine Pseudonymisierungseinheit (100) zu senden, wobei der Befehl (Be) Steueranweisungen enthält, um zu der gerade dargestellten Bilddatenstudie (BDS') eine Vorgängerstudie (BDS-V) von dem Bildsendeknoten (10) zu laden,
- Die Pseudonymisierungseinheit (100) auf dem Bildsendeknoten (10), umfassend:
- Einen Extraktor (102), der dazu bestimmt ist, auf Empfang des Befehls (Be) einen Identifikationsdatensatz (ID) aus dem Befehl (Be) zu extrahieren
- Einen Lader (104), der dazu bestimmt ist, auf Basis des extrahierten Identifikationsdatensatzes (ID) auf das Bildspeichersystem (PACS) zum Laden der Vorgängerstudie (BDS-V) zu zugreifen , wobei die Vorgängerstudie (BDS-V) einen Bilddatensatz umfasst
- Einen Pseudonymisierungsprozessor (106), der dazu bestimmt ist, zu der geladenen Vorgängerstudie (BDS-V) einen pseudonymisierten Bilddatensatz (BDS-V') basierend auf dem Bilddatensatz der geladenen Vorgängerstudie (BDS-V) zu erzeugen,
- Eine Empfängerknoten-Schnittstelle (110), die dazu bestimmt ist, den pseudonymisierten Bilddatensatz (BDS-V`) an den Empfängerknoten (20) zu senden.

2. System nach dem vorhergehenden Patentanspruch 1, bei dem die Pseudonymisierungseinheit (100) ein Pseudonymisierungs-Frontend (100-F) und ein Pseudonymisierungs-Backend (100-B) umfasst, wobei das Pseudonymisierungs-Frontend (100-F) auf dem Bildsendeknoten (10) und das Pseudonymisierungs-Backend (100-B) entfernt von dem Bildsendeknoten (10) angeordnet ist.

3. System nach einem der vorhergehenden Systemansprüche, bei dem die Befehlseinheit (200) ein Befehls-Frontend (200-F) und ein Befehls-Backend (200-B) umfasst, wobei das Befehls-Frontend (200-F) auf dem Empfängerknoten (20) und das Befehls-Backend (200-B) entfernt von dem Empfängerknoten (20) angeordnet sind.

4. System nach einem der vorhergehenden Systemansprüche, bei dem die Befehlseinheit (200) und die Pseudonymisierungseinheit (100) über ein Netzwerk (NW), insbesondere über ein öffentliches Netzwerk, in Datenaustausch stehen.

5. Verfahren zum gesicherten Nachladen von Vorgängerstudien (BDS-V) zu einer Bilddatenstudie (BDS) auf einem Empfängerknoten (20), der entfernt von einem Bildsendeknoten (10) angeordnet ist, wobei das Verfahren folgende Verfahrensschritte umfasst:
- Anzeigen eines Signalsensors (202) auf dem Empfängerknoten (20) zeitgleich mit der gerade dargestellten Bilddatenstudie (BDS') auf dem Empfängerknoten (10)
- Erfassen eines Bildanforderungssignals (S1) auf dem Signalsensor (202) des Empfängerknotens (20)
- Nach Erfassen des Bildanforderungssignals (S1) Erzeugen eines Befehls (Be) durch eine Befehlseinheit (200) des Empfängerknotens (20), wobei der Befehl (Be) Steueranweisungen enthält, um zu der gerade dargestellten Bilddatenstudie (BDS') eine Vorgängerstudie (BDS-V) von dem Bildsendeknoten (10) zu laden,
- und Versenden des Befehls (Be) an eine Pseudonymisierungseinheit (100) des Bildsendeknoten (10) durch den Empfängerknoten (20)
- Empfangen des Befehls (Be) der Befehlseinheit (200) auf der Pseudonymisierungseinheit (100)
- Extrahieren eines Identifikationsdatensatzes (ID) aus dem Befehl (Be) durch die Pseudonymisierungseinheit (100)
- Laden der Vorgängerstudie (BDS-V) von einem Bildspeichersystem (PACS) auf Basis des extrahierten Identifikationsdatensatzes (ID) durch die Pseudonymisierungseinheit (100), wobei die Vorgängerstudie (BDS-V) einen Bilddatensatz umfasst
- Erzeugen eines pseudonymisierten Bilddatensatzes zu der Vorgängerstudie (BDS-V') basierend auf dem Bilddatensatz der geladenen Vorgängerstudie (BDS-V) durch die Pseudonymisierungseinheit (100),
- Versenden des erzeugten pseudonymisierten Bilddatensatzes zu der Vorgängerstudie (BDS-V') an den Empfängerknoten (20) durch die Pseudonymisierungseinheit (100).

6. Verfahren nach dem vorstehenden Verfahrensanspruch, bei dem eine Zuordnung zwischen Bilddatensatz (BDS), Identifikationsdatensatz (ID) und/oder pseudonymisiertem Bilddatensatz zu der Vorgängerstudie (BDS-V`) gespeichert wird.

7. Verfahren nach dem vorstehenden Verfahrensanspruch, bei dem die Zuordnung auf der Pseudonymisierungseinheit (100) gespeichert wird.

8. Verfahren nach einem der vorhergehenden Verfahrensansprüche, bei dem der Befehl (Be) einen Identifikationsdatensatz (ID) umfasst.

9. Verfahren nach dem vorstehenden Verfahrensanspruch, bei dem der Identifikationsdatensatz (ID) den Patienten und/oder die Bildstudie (BDS) eineindeutig identifiziert.

10. Verfahren nach einem der vorhergehenden Verfahrensansprüche, bei dem das Bildanforderungssignal (S1) nur zu einem angezeigten Bilddatensatz (BDS`) eingegeben werden kann, indem auf einer Bildschirmoberfläche des Empfängerknotens (20) ein Eingabefeld nur während der Darstellung eines Bilddatensatzes bereitgestellt wird.

11. Computerprogrammprodukt, das in einen internen Speicher eines digitalen Computers geladen werden kann und Softwareroutinen umfasst, mit denen die Schritte des Verfahrens gemäß den vorstehenden Verfahrensansprüchen ausgeführt werden, wenn die Softwareroutinen auf dem jeweiligen digitalen Computer ausgeführt werden.

## Claims

1. System for secure reloading of previous studies (BDS-V) relating to an image data study (BDS) on a receiver node (20) from a remote image sending node (10), comprising:
- the receiver node (20) for representing image data studies, which comprises a command unit (200) with a signal sensor (202), wherein the signal sensor (202) is indicated at the same time as the image data study (BDS') currently shown and is designed to capture an image request signal (S1) relating to the image data study (BDS') currently shown and wherein the command unit (200) is designed, after capturing the respective image request signal (S1), to generate a command (Be) and to send the same to a pseudonymisation unit (100), wherein the command (Be) contains control instructions, in order to load a previous study (BDS-V) from the image sending node (10) relating to the image data study (BDS') currently shown,
- the pseudonymisation unit (100) on the image sending node (10), comprising:
- an extractor (102), which is designed to extract an identification data set (ID) from the command (Be) upon receipt of the command (Be),
- a loader (104), which is designed to access the image storage system (PACS) for loading the previous study (BDS-V) on the basis of the extracted identification data set (ID), wherein the previous study (BDS-V) comprises an image data set,
- a pseudonymisation processor (106), which is designed to generate a pseudonymised image data set (BDS-V') based on the image data set of the loaded previous study (BDS-V) relating to the loaded previous study (BDS-V),
- a receiver node interface (110), which is designed to send the pseudonymised image data set (BDS-V') to the receiver node (20).

2. System according to the preceding claim 1, in which the pseudonymisation unit (100) comprises a pseudonymisation frontend (100-F) and a pseudonymisation backend (100-B), wherein the pseudonymisation frontend (100-F) is arranged on the image sending node (10) and the pseudonymisation backend (100-B) is arranged remote from the image sending node (10).

3. System according to one of the preceding system claims, in which the command unit (200) comprises a command frontend (200-F) and a command backend (200-B), wherein the command frontend (200-F) and the command backend (200-B) are arranged on the receiver node (20) and remote from the receiver node (20) in each instance.

4. System according to one of the preceding system claims, in which the command unit (200) and the pseudonymisation unit (100) exchange data with one another by way of a network (NW), in particular by way of a public network.

5. Method for secure reloading of previous studies (BDS-V) relating to an image data study (BDS) on a receiver node (20), which is arranged remote from an image sending node (10), wherein the method comprises the following method steps:
- indicating a signal sensor (202) on the receiver node (20) at the same time as the image data study (BDS') currently shown on the receiver node (10),
- capturing an image request signal (S1) on the signal sensor (202) of the receiver node (20),
- after capturing the image request signal (S1), generating a command (Be) by means of a command unit (200) of the receiver node (20), wherein the command (Be) contains control instructions in order to load a previous study (BDS-V) from the image sending node (10) relating to the image data study (BDS') currently shown,
- and sending the command (Be) to a pseudonymisation unit (100) of the image sending node (10) by means of the receiver node (20),
- receiving the command (Be) of the command unit (200) on the pseudonymisation unit (100)
- extracting an identification dataset (ID) from the command (Be) by means of the pseudonymisation unit (100)
- loading the previous study (BDS-V) from an image storage system (PACS) on the basis of the extracted identification data set (ID) by means of the pseudonymisation unit (100), wherein the previous study (BDS-V) comprises an image data set,
- generating a pseudonymised image data set relating to the previous study (BDS-V') on the basis of the image data set of the loaded previous study (BDS-V) by means of the pseudonymisation unit (100),
- sending the generated pseudonymised image data set relating to the previous study (BDS-V') to the receiver node (20) by means of the pseudonymisation unit (100).

6. Method according to the preceding method claim, in which an assignment between image data set (BDS), identification data set (ID) and/or pseudonymised image data set is stored relating to the previous study (BDS-V').

7. Method according to the preceding method claim, in which the assignment is stored on the pseudonymisation unit (100).

8. Method according to one of the preceding method claims, in which the command (Be) comprises an identification data set (ID) .

9. Method according to the preceding method claim, in which the identification data set (ID) identifies the patient and/or the image study (BDS) one-to-one.

10. Method according to one of the preceding method claims, in which the image request signal (S1) can only be input relating to an indicated image data set (BDS') by an input field only being provided during the display of the image data set on an image screen of the receiver node (20).

11. Computer program product which can be loaded into an internal memory of a digital computer and comprises software routines, with which the steps of the method according to the preceding method claims are executed, when the software routines are executed on the respective digital computer.

## Revendications

1. Système de post-chargement sécurisé d'études (BDS-V) précurseures par rapport à une étude (BDS) de données d'image sur un noeud (20) récepteur par un noeud (10) émetteur d'image à distance, comprenant :
- le noeud (20) récepteur pour la représentation d'études de données d'image, qui comprend une unité (200) de commandement ayant un capteur (202) de signal, dans lequel le capteur (202) de signal est affiché en même temps que l'étude (BDS') de données d'image représentée précisément et est destiné à détecter un signal (S1) de demande d'image par rapport à l'étude (BDS') de données d'image représentée précisément et dans lequel l'unité (200) de commandement est destinée, après détection du signal (S1) de demande d'image respectif, à produire un commandement (Be) et à l'envoyer à une unité (100) de pseudonymisation, dans lequel le commandement (Be) contient des instructions de commande pour charger, par le noeud (10) émetteur d'image, par rapport à l'étude (BDS') de données d'image représentée précisément, une étude (BDS-V) précurseure
- l'unité (100) de pseudonymisation sur le noeud (10) émetteur d'image, comprenant :
- un extracteur (102), qui est destiné, à réception du commandement (Be), à extraire du commandement (Be) un ensemble (ID) de données d'identification,
- un chargeur (104), qui est destiné à accéder, sur la base de l'ensemble (ID) de données d'identification extrait, au système (PACS) de mémoire d'image pour le chargement de l'étude (BDS-V) précurseure, dans lequel l'étude (BDS-V) précurseure comprend un ensemble de données d'image,
- un processeur (106) de pseudonymisation, qui est destiné à produire, par rapport à l'étude (BDS-V) précurseure chargée, un ensemble (BDS-V') de données d'image pseudonymisé sur la base de l'ensemble de données d'image de l'étude (BDS-V) précurseure chargée,
- une interface (110) de noeud récepteur, qui est destinée à envoyer l'ensemble (BDS-V') de données d'image peudonymisé au noeud (20) récepteur.

2. Système suivant la revendication 1 précédente, dans lequel l'unité (100) de pseudonymisation comprend une extrémité (100-F) avant de pseudonymisation et une extrémité (100-B) arrière de pseudonymisation, dans lequel l'extrémité (100-F) avant de pseudonymisation est disposée sur le noeud (10) émetteur d'image et l'extrémité (100-B) arrière de pseudonymisation est disposée à distance du noeud (10) émetteur d'image.

3. Système suivant l'une des revendications de système précédentes, dans lequel l'unité (200) de commandement comprend une extrémité (200-F) avant de commandement et une extrémité (200-B) arrière de commandement, dans lequel l'extrémité (200-F) avant de commandement est disposée sur le noeud (20) récepteur et l'extrémité (200-B) arrière de commandement est disposée en étant à distance du noeud (20) récepteur.

4. Système suivant l'une des revendications de système précédente, dans lequel l'unité (200) de commandement et l'unité (100) de pseudonymisation sont en échange de données en passant par un réseau (NW), en particulier par un réseau public.

5. Procédé de post-chargement sécurisé d'études (BDS-V) précurseures par rapport à une étude (BDS) de données d'image sur un noeud (20) récepteur, qui est disposé à distance d'un noeud (10) émetteur d'image, dans lequel le procédé comprend les stades de procédé suivants :
- affichage d'un capteur (202) de signal sur le noeud (20) récepteur en même temps que l'étude (BDS') de données d'image représentée précisément sur le noeud (10) récepteur,
- détection d'un signal (S1) de demande d'image sur le capteur (202) de signal du noeud (20) récepteur,
- après détection du signal (S1) de demande d'image, production d'un commandement (Be) par une unité (200) de commandement du noeud (20) récepteur, dans lequel le commandement (Be) contient des instructions de commande pour charger, par le noeud (10) émetteur d'image, par rapport à l'étude (BDS') de données d'image représentée précisément, une étude (BDS-V) précurseure,
- et envoi du commandement (Be) à une unité (100) de pseudonymisation du noeud (10) émetteur d'image par le noeud (20) récepteur,
- réception du commandement (Be) de l'unité (200) de commandement sur l'unité (100) de pseudonymisation,
- extraction, par l'unité (100) de pseudonymisation, d'un ensemble (ID) de données d'identification du commandement (Be),
- chargement, par l'unité (100) de pseudonymisation, de l'étude (BDS-V) précurseure d'un système (PACS) de mémoire d'image sur la base de l'ensemble (ID) de données d'identification extrait, dans lequel l'étude (BDS-V) précurseure comprend un ensemble de données d'image,
- production, par l'unité (100) de pseudonymisation, d'un ensemble de données d'image pseudonymisé par rapport à l'étude (BDS-V') précurseure sur la base de l'ensemble de données d'image de l'étude (BDS-V) précurseure chargée,
- envoi, par l'unité (100) de pseudonymisation, de l'ensemble de données d'image pseudonymisé, produit par rapport à l'étude (BDS-V') précurseure, au noeud (20) récepteur.

6. Procédé suivant la revendication de procédé précédente, dans lequel on met en mémoire une association entre un ensemble (BDS) de données d'image, un ensemble (ID) de données d'identification et/ou un ensemble de données d'image pseudonymisé et à l'étude (BDS-V') précurseure.

7. Procédé suivant la revendication de procédé précédente, dans lequel on met en mémoire l'association sur l'unité (100) de pseudonymisation.

8. Procédé suivant l'une des revendications de procédé précédentes, dans lequel le commandement (Be) comprend un ensemble (ID) de données d'identification.

9. Procédé suivant la revendication de procédé précédente, dans lequel l'ensemble (ID) de données d'identification identifie de manière univoque le patient et/ou l'étude (BDS) d'image.

10. Procédé suivant l'une des revendications de procédé précédentes, dans lequel le signal (S1) de demande d'image ne peut être entré que par rapport à un ensemble (BDS') de données d'image affiché par le fait que l'on dispose, sur une surface d'écran du noeud (20) récepteur, d'un champ d'entrée seulement pendant la représentation d'un ensemble de données d'image.

11. Produit de programme d'ordinateur, qui peut être chargé dans une mémoire interne d'un ordinateur numérique et qui comprend des routines de logiciels, par lesquelles les stades suivant les revendications de procédé précédentes sont exécutés lorsque les routines de logiciels sont exécutées sur l'ordinateur numérique respectif.
